Europäisches Patentamt

European Patent Office

Office européen des brevets

(19)

(11) Veröffentlichungsnummer: **0 337 420**
**A2**

(12) **EUROPÄISCHE PATENTANMELDUNG**

(21) Anmeldenummer: **89106497.4**

(22) Anmeldetag: **12.04.89**

(51) Int. Cl.4: **A61M 1/28** , **A61L 2/18**

(30) Priorität: **15.04.88 DE 3812524**

(43) Veröffentlichungstag der Anmeldung:
**18.10.89 Patentblatt 89/42**

(84) Benannte Vertragsstaaten:
**AT BE CH DE ES FR GB IT LI NL SE**

(71) Anmelder: **Fresenius AG**
**Gluckensteinweg 5**
**D-6380 Bad Homburg v.d.H.(DE)**

(72) Erfinder: **Steudle, Volker, Dr.**
**Rohnstrasse 26**
**D-6300 Giessen(DE)**
Erfinder: **Bartz, Volker**
**Ludwig-Richter-Stasse 24**
**D-6300 Giessen(DE)**

(74) Vertreter: **Görtz, Dr. Fuchs, Dr. Luderschmidt**
**Patentanwälte**
**Abraham-Lincoln-Strasse 7 Postfach 46 60**
**D-6200 Wiesbaden(DE)**

(54) **Dialysier- und Spüllösung für die intraperitoneale Verabreichung mit antimikrobieller Ausrüstung.**

(57) Peritonealdialysierflüssigkeit mit Elektrolyten in physiologischen Mengen und wenigstens einer osmotisch wirksamen Substanz, die zusätzlich Sorbinsäure als antimikrobielle Substanz in einer Menge von 0,001 bis 1 Gew.-% und einen pH-Wert von 4 bis 7 aufweist.

EP 0 337 420 A2

## Dialysier- und Spüllösung für die intraperitoneale Verabreichung mit antimikrobieller Ausrüstung

Gegenstand der vorliegenden Erfindung ist eine Dialysier-und Spülflüssigkeit zur intraperitonealen Verabreichung, die Elektrolyte in physiologischen Mengen und wenigstens eine osmotisch wirksame Substanz enthält, sowie die Verwendung von Sorbinsäure als antimikrobiell wirksame Substanz bei Dialysier- und Spülflüssigkeiten für die intraperitoneale Verabreichung bzw. die Verwendung von Sorbinsäure als antimikrobielle Substanz zur Herstellung einer Dialysier- und Spülflüssigkeit für die intraperitoneale Verabreichung.

Bei der sogenannten CAPD (kontinuierlichen ambulanten Peritonealdialyse) wird das Peritoneum von nierenkranken Patienten mehrfach täglich mit einer frischen Peritonealdialysierflüssigkeit gefüllt, wobei zuvor verbrauchte Peritonealdialysierflüssigkeit durch einen Katheter nach außen abgelassen wird. Hierzu wird mittels eines besonders geschützten Konnektors, der sich am Ende eines Intraperitonealkatheters befindet, jeweils ein Beutel mit frischer Dialysierflüssigkeit angeschlossen, nachdem ein die verbrauchte Dialysierflüssigkeit enthaltender Beutel vom Konnektor abgetrennt worden ist. Dieser Konnektionsvorgang muß unter absolut sterilen Bedingungen durchgeführt werden, da ansonsten die Gefahr der Einschleppung von Keimen durch den geöffneten Konnektor besteht. Trotz intensiver Schulung der Patienten und trotz aller erdenklichen Maßnahmen zur Aufrechterhaltung der Sterilität stellen sich immer wieder Entzündungen des Peritoneums, d.h. eine Peritonitis ein, die somit die Hauptkomplikation der Peritonealdialyse darstellt. Sie tritt - wie vorstehend festgestellt - meist intraluminal durch extrakorporale Kontamination oder kanalikular durch Tunnelinfektion auf, die durch Einschleusung von Keimen im Tunnel bereich des Intraperitonealkatheters entstehen. Dabei machen die infektiösen Peritonitiden den überwiegenden Teil der bei der Peritonealdialyse auftretenden Peritonitiden aus, die nur unter massivem Einsatz von Antibiotika behandelbar sind und im übrigen den Abbruch der CAPD und den Anschluß des Patienten an eine Hämodialysemaschine erzwingen.

Zur Vermeidung der Keimeinschleusung bzw. zur erheblichen Verringerung der Peritonitisgefahr wurden speziell ausgebildete Konnektoren vorgeschlagen, bei denen die Konnektionsbereiche nicht mehr berührbar sind (DE-PS 28 53 635). Des weiteren wurden vorgeformte Intraperitonealkatheter vorgeschlagen, um den Spannungsdruck des Katheters im Implantatkanal zu beseitigen (DE-PS 31 47 722). Ferner wurden Aufheizvorrichtungen zur Sterilisierung des zusammengesteckten Konnektors vorgeschlagen (DE-OS 36 01 893). Schließlich wurden spezielle Konnektoren vorgeschlagen, die vor der Einleitung der Flüssigkeit in das Peritoneum ein Durchspülen des Konnektors und somit das Ausschwemmen von Keimen ermöglichen (DE-OSen 35 13 204 und 35 13 205).

Dennoch ist die Gefahr einer Peritonitis bei der CAPD immer noch nicht endgültig abgewendet worden.

Eine derartige Peritonitis wird am häufigsten durch Staphylococcus epidermidis mit blanderem Verlauf und durch Staphylococcus aureus mit schwerem und langwierigem Verlauf erregt. Die Infektionswege sind bei diesen Erregern normalerweise transluminal durch den geöffneten Konnektor und Katheter, da 3 bis 4 mal pro Tag beim Dialysatwechsel ein Eindringen von Keimen durch Luft, Staub, Tröpfchen und durch direkte Berührung der Konnektorinnenseite durch die Öffnung des Systems möglich ist.

Neben dem Eindringen von Keimen entlang des Katheters aufgrund eines Tunnelinfektes werden Infektionen über perforierte oder entzündete Organe im Bauchraum sowie aufsteigende Infektionen aus dem Genitaltrakt der Frau durch die Tuben beobachtet. Des weiteren können hämatogene Streuungen vorkommen. Schließlich spielen bei postoperativen diffusen Peritonitiden Pilzinfektionen mit Candida albicans als häufigster Erreger eine Rolle.

Für die intraperitoneal eingeschleusten Mikroorganismen existieren ideale Wachstums-(Vermehrungs)-bedingungen, zumal die eingesetzten Spüllösungen nahezu ausschließlich Glucose als osmotisch wirksame Substanz aufweisen, die hervorragende Wachstumsbedingungen für Mikroben garantiert. Ziel einer Peritonitisprophylaxe ist es also, ein Eindringen von Mikroorganismen in die Peritonealhöhle zu verhindern oder aber deren Wachstum intraperitoneal zu inhibieren.

Der Erfindung liegt daher die Aufgabe zugrunde, eine Dialyse- und Spüllösung der eingangs erwähnten Art zur Verfügung zu stellen, die über längere Zeit an Patienten intraperitoneal verabreicht werden kann und eine infektiöse Pertonitiskomplikation möglichst weitgehend vermindert, wenn nicht sogar verhindert.

Diese Aufgabe wird durch eine Dialysier- und Spülflüssigkeit gelöst, die durch einen antimikrobiellen Gehalt an Sorbinsäure und einen pH-Wert von 4 bis 7 gekennzeichnet ist.

Überraschenderweise hat sich nunmehr ergeben, daß der Einsatz von Sorbinsäure in Peritonealdialysierflüssigkeiten das Wachstum von in die Peritonealhöhle eingeschleppten Keimen derart hemmt, daß die körpereigenen Abwehrkräfte erfolgreich zum Zuge kommen können, d.h. daß Peritonitiden wirksam verhindert werden können.

EP 0 337 420 A2

Bei der Sorbinsäure, die die Formel

$$CH_3-CH = CH-CH = CH-COOH$$

hat, handelt es sich um die 4,2-Hexadiensäure oder 2-Propylenacrylsäure mit der Summenformel $C_6H_8O_2$

Die Sorbinsäure weist eine antimikrobielle Wirkung auf, die üblicherweise auf der nichtdissoziierten lipoidlöslichen Form beruht. Insofern ist also der Einsatz von Sorbinsäure pH-abhängig.

Die Sorbinsäure wird bekanntermaßen im Lebensmittelbereich zur Konservierung von Lebensmitteln und weiteren Bedarfsgegenständen eingesetzt. Gemäß Seiten 841 bis 843 des Lexikon der Hilfsstoffe für Pharmazie, Kosmetik und angrenzende Gebiete, H. Fiedler, 1981, Bd. 9 (OVR Oberschwäbische Verlagsanstalt Ravensburg), wird Sorbinsäure als Konservierungsmittel für pharmazeutische und kosmetische Präparate empfohlen sowie deren Verwendung zur Konservierung von Lebensmitteln beschrieben. In der DE-AS 1 198 659 werden spezielle, Sorbitylreste aufweisende gemischte Anhydride, wie Sorbinsäure-ethyl-kohlensäureanhydrid, Sorbinsäure-essigsäureanhydrid und Propionsäuresorbinsäureanhydrid als Konservierungsmittel zur Haltbarmachung verderblicher Stoffe vegetabilischer oder animalischer Herkunft, wie Lebensmitteln, Injektionslösungen und anderen pharmazeutischen oder kosmetischen Zubereitungen vorgeschlagen, wobei die mikrobizide Wirkung dieser Anhydride der von Sorbinsäure überlegen sein soll.

Erfindungsgemäß ist der Einsatz von Sorbinsäure im schwachsauren Bereich (pH 5 bis 6) bevorzugt, wobei der Einsatz jedoch nicht ausschließlich auf diesen bevorzugten Bereich beschränkt ist. So sind auch pH-Werte von 4 bis 7 als anwendbar zu bezeichnen, wobei ein pH-Wert von 4 unter Umständen bereits zu Irritationen der Schleimhaut des Peritoneums führen kann und der pH-Wert von 7 auf grund der sehr schwachen Dissoziation (nur etwa 0,7 % der Sorbinsäure liegen undissoziiert vor) eine erhöhte Ausgangskonzentration von Sorbinsäure notwendig macht.

Erfindungsgemäß werden in der Peritonealdialysierflüssigkeit 0,001 bis 1 Gew.-% Sorbinsäure eingesetzt, wobei unter Sorbinsäure erfindungsgemäß der undissoziierte Bestandteil zu verstehen ist. Dieser Bestandteil läßt sich aufgrund des zum Einsatz kommenden pH-Wertes und der pK-Konstante der Sorbinsäure von $1,73 \times 10^{-5}$ ohne Probleme errechnen.

Neben der reinen Sorbinsäure sind daher auch ihre Salze mit Basen, beispielsweise Natrium-, Kaliumoder Calciumsorbat einsetzbar, die nach Auflösung in Wasser und Einstellung des pH-Wertes mit Hilfe von Säuren (Salzsäure) in den entsprechenden nichtdissoziierten Bestandteil der Sorbinsäure und die restlichen dissoziierten Bestandteile zerfallen.

Wie bereits vorstehend festgestellt, wird Sorbinsäure in einem weiten Bereich zur Konservierung von Lebensmitteln eingesetzt. Sorbinsäure ist eine Fettsäure und wird daher im menschlichen Organismus wie jede andere Fettsäure vollständig verstoffwechselt. Insofern ist eine hervorragende Biokompatibilität bei dem Einsatz von Sorbinsäure in Peritonealdialysierflüssigkeiten gewährleistet. Es besteht daher nicht das Problem der Einlagerung von Sorbinsäure in den menschlichen Organismus aufgrund einer schweren Metabolisierbarkeit, wie dies beispielsweise bei Zuckeraustauschstoffen (Sorbit, Xylit) oder den polymeren Zuckern der Fall ist.

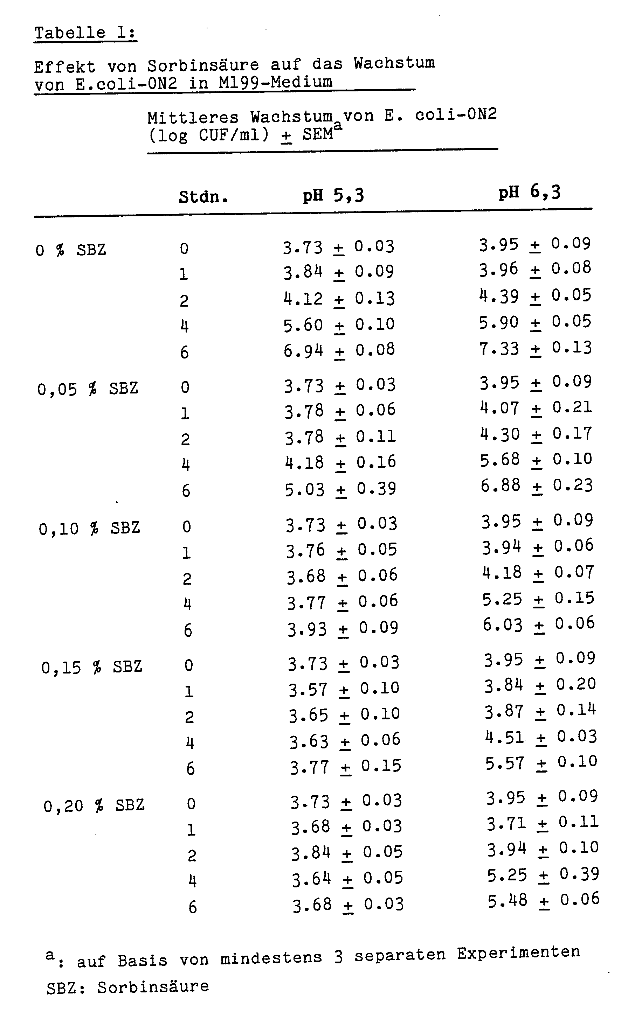

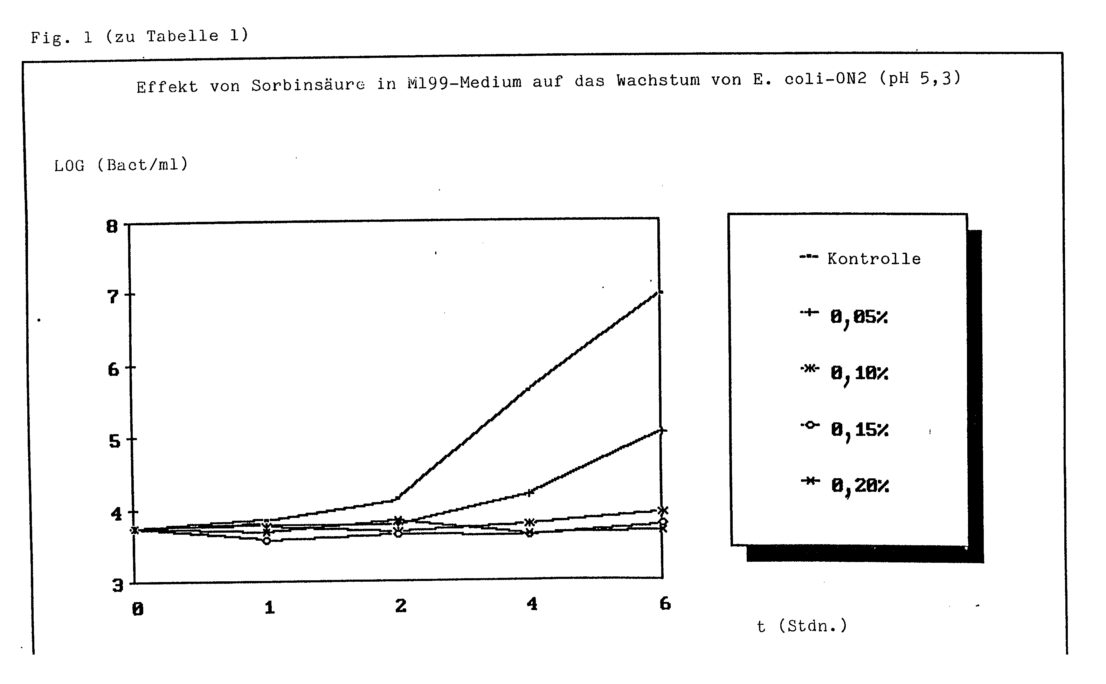

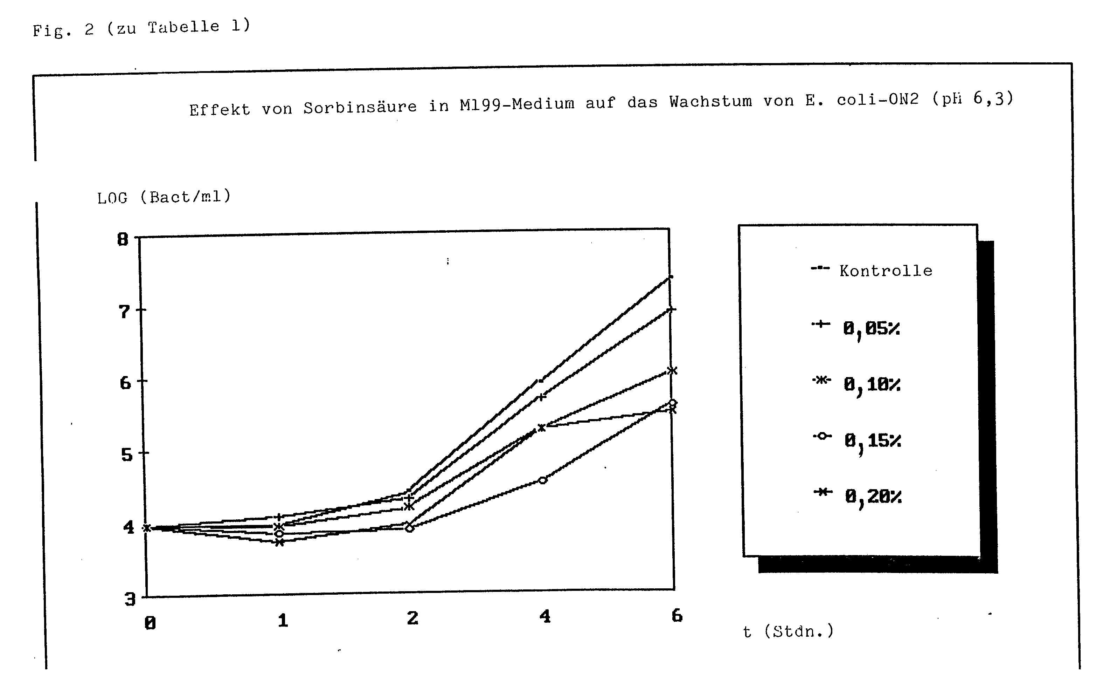

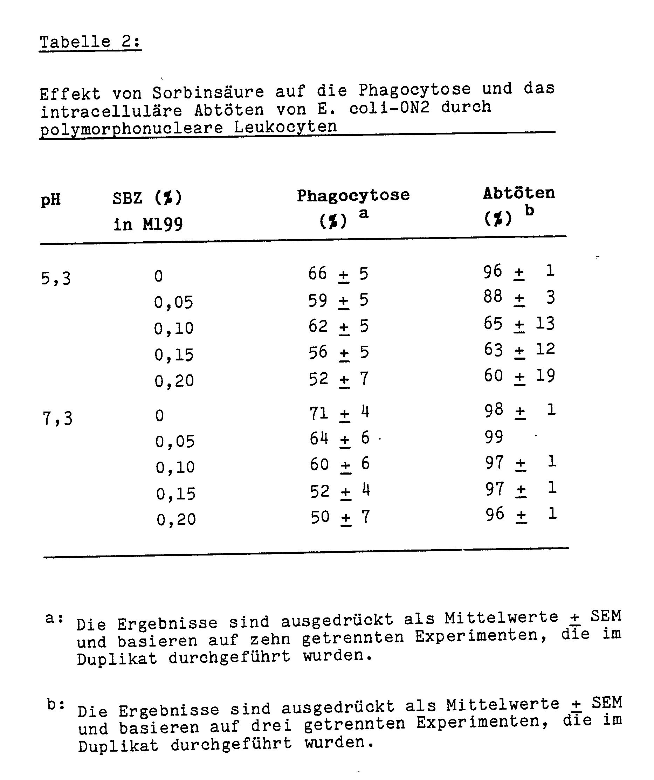

Die Sorbinsäure weist in Abhängigkeit von der eingesetzten Konzentration eine gute fungistatische bzw. fungizide Wirkung auf und vermag beispielsweise Mikroorganismen, wie Candida albicans relativ gut in ihrem Wachstum zu hemmen bzw. abzutöten. Des weiteren ist auch die bakteriostatische bzw. bakterizide Wirkung wenigstens bei einem pH-Wert von 6 ebenfalls gut. So können Mikroorganismen, wie Staphylococcus aureus, Klebsiella pneumoniae, E. coli und dgl., bereits mit geringen Hemmkonzentrationen an ihrem Wachstum gehemmt werden (vgl. Fig. 1 und 2)

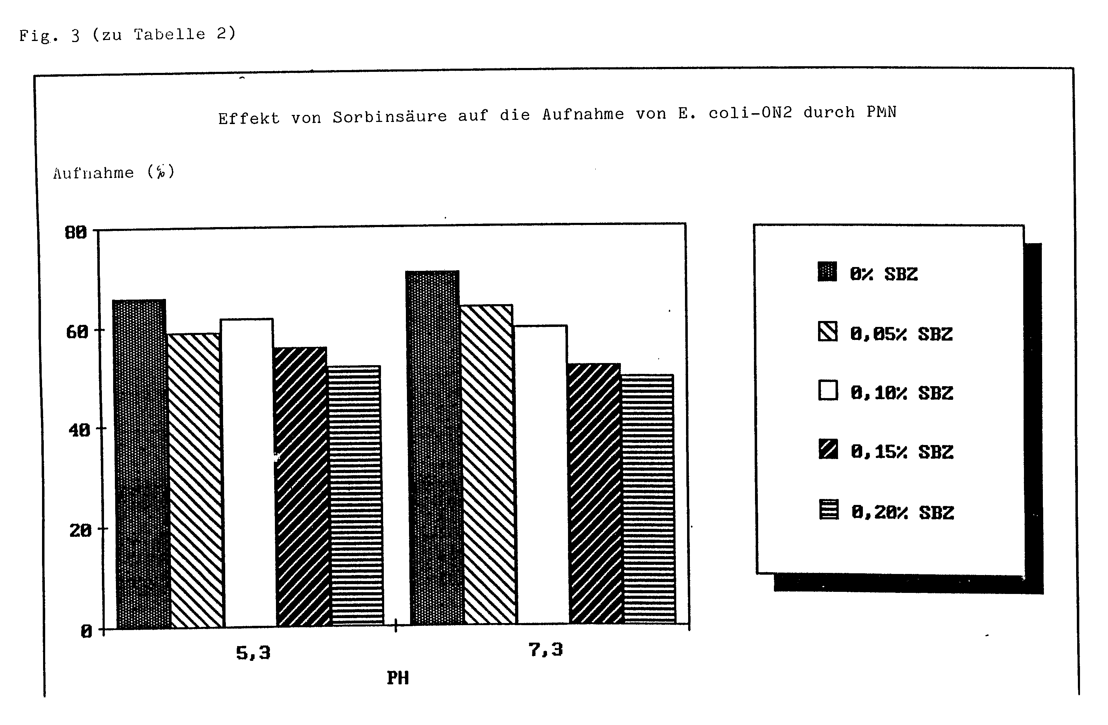

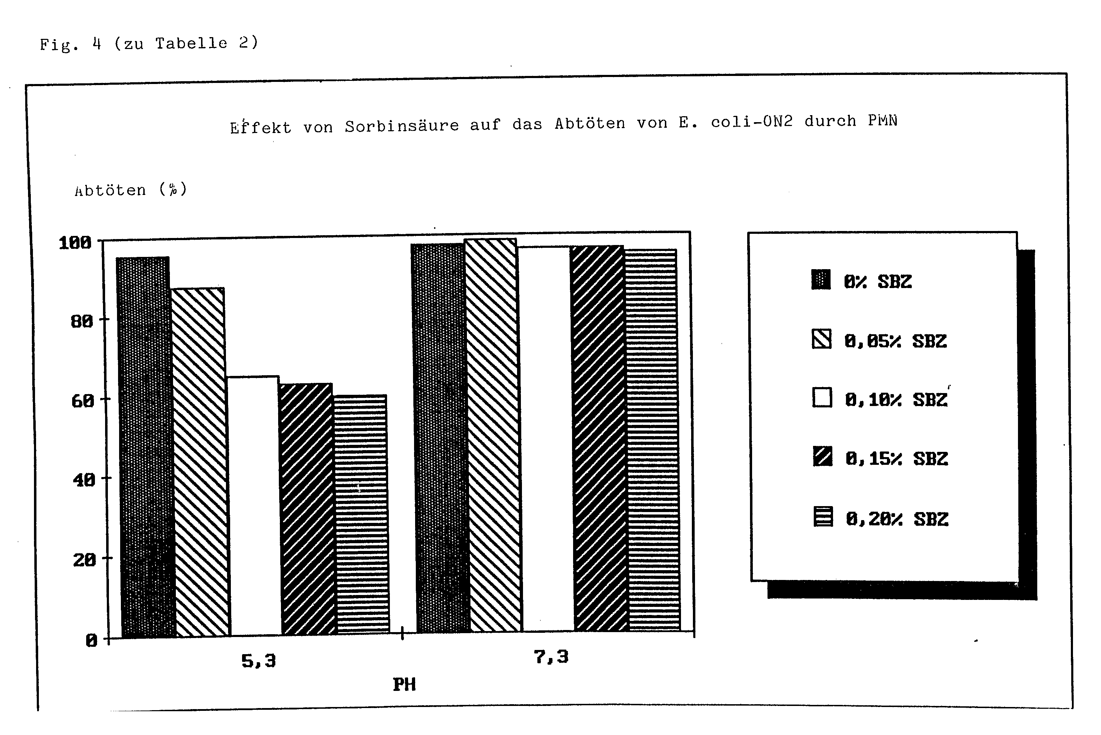

Gewöhnlich reicht es aus, wenn die Sorbinsäure in einer solchen Konzentration vorliegt, daß das Wachstum gehemmt wird, da der Körper beispielsweise eine Keimmenge von 100 ohne weiteres mit den eigenen Abwehrmechanismen, beispielsweise durch Makrophagen, beherrschen kann. Durch die Verwendung der Sorbinsäure wird der eigene Abwehrmechanismus, zum Beispiel durch Makrophagen oder polymorphonucleare Leukozyten, praktisch nicht (bei höherem pH-Wert) bzw. nur in geringem Umfang (bei niederem pH-Wert, zum Beispiel pH 5,3) reduziert. Das bedeutet, daß bei der erfindungsgemäßen Verwendung von Sorbinsäure die eigenen Abwehrmechanismen nach wie vor wirksam sind (vgl. Fig. 3 und 4).

Der vorstehend genannte pH-Wert der erfindungsgemäßen Dialysierflüssigkeit läßt sich mit Hilfe von Milchsäure oder Essigsäure in Verbindung mit Natronlauge einstellen, wobei die Milchsäure bevorzugt ist. Vorzugsweise weist die Dialysierflüssigkeit etwa einen pH-Wert von 5,6 bei einer Lactatkonzentration von 35 mmol/l auf.

Es sind jedoch aber auch andere übliche organische oder anorganische Säuren, sofern sie vom menschlichen Körper akzeptiert werden, zur Einstellung des pH-Wertes einsetzbar.

Die Sorbinsäure ist ein leicht erhältliches billiges Handelsprodukt. Sie weist üblicherweise einen Reinheitsgrad von über 99 % auf. Des gleichen kann sie unproblematisch hitzesterilisiert werden, d.h. auf die übliche Sterilisationstemperatur von 115 bis 122 °C erwärmt werden.

Die erfindungsgemäße Dialysier- und Spülflüssigkeit besteht aus einer Zusammensetzung von auf dem Markt erhältlichen oder sonstigen in der Literatur erwähnten Lösungen, mit der Ausnahme, daß diesen Lösungen die Sorbinsäure oder deren Salze als antimikrobielle Substanz in der vorstehend erwähnten Konzentration von 0,001 bis 1 Gew.-% zugesetzt werden.

Die auf dem Markt befindlichen Lösungen enthalten, um physiologisch unbedenklich zu sein, Elektrolytsalze mit physiologischem Pegel.

Die Ionenkonzentrationen in der erfindungsgemäßen Dialysier- und Spülflüssigkeit betragen vorteilhafterweise 125 bis 150, insbesondere 132 bis 140 mmol/l Na$^+$; 0 bis 8, insbesondere 0 bis 4 mmol/l K$^+$; 0 bis 3, insbesondere 0,5 bis 2 mmol/l Ca$^{++}$; 0 bis 2,5, insbesondere 0,3 bis 1 mmol/l Mg$^{++}$; 10 bis 60, insbesondere 30 bis 50 mmol/l Ionen, ausgewählt aus der Gruppe Lactat-, Acetat- und Bicarbonat-Ionen und Rest Cl$^-$.

Des weiteren enthält die erfindungsgemäße Dialysier- und Spülflüssigkeit eine osmotisch wirksame Substanz in osmotisch wirksamen Mengen. Im weit überwiegenden Maß wird Glucose als osmotisch wirksame Substanz eingesetzt, wobei der Einsatz einer solchen Substanz nicht vom Erfindungsgedanken erfaßt wird. Insofern sind auch alle anderen derzeit erörterten Substanzen als osmotisch wirksame Substanz einsetzbar. Bezug genommen wird auf die am gleichen Tag von der gleichen Anmelderin eingereichte Patentanmeldung mit dem Titel "Dialysier- und Spüllösung zur intraperitonealen Verabreichung" (P 38 12 525.0-35, unser Zeichen FR 1367), die den Einsatz von Galactose als osmotisch wirksame Substanz vorschlägt und im übrigen weitere osmotisch wirksame Substanzen erläutert.

Diese Substanzen kommen in osmotisch wirksamen Mengen vor, so daß der osmotische Druck zwischen 300 bis 700, insbesondere 320 bis 550 und vorzugsweise 350 bis 450 mosm/l beträgt.

So weist eine Peritonealdialyselösung mit 16,5 g bzw. 46,76 g/l Galactosehydrat einen theoretischen osmotischen Druck von 355 bzw. 507 mosm/l auf, der im übrigen durch die im Verhältnis hierzu geringe Menge Sorbinsäure etwas erhöht wird.

Des gleichen können auch übliche Zusatzstoffe, beispielsweise Insulin u. dgl. in der erfindungsgemäßen Dialyselösung zum Einsatz kommen. Auch dieser Einsatz ist nicht vom erfindungsgemäßen Gedanken umfaßt.

Das nachfolgende Beispiel erläutert die Erfindung.

Beispiel

In 1 l Wasser von Injektionsqualität wird eine Lösung von Glucose als osmotisch wirksame Substanz, Elektrolytsalzen in Form des Lactats, Acetats oder Chlorids und Sorbinsäure oder deren Salzen hergestellt und anschließend auf einen Beutel abgefüllt, der hitzesterilisiert wird.

Die Lösung weist folgende Zusammensetzung auf:

| | | |
|---|---|---|
| Na$^+$ | 132,00 | mmol/l |
| Ca$^{2+}$ | 1,75 | mmol/l |
| Mg$^{2+}$ | 0,75 | mmol/l |
| Cl$^-$ | 102,00 | mmol/l |
| Lactat | 35,0 | mmol/l |
| Glucose | 16,5 | g/l |
| Sorbinsäure | 1,0 | g/l |

Der theoretische osmotische Druck beträgt 355 mosm/l, während die Sorbinsäurekonzentration bei 0,1 Gew.-% liegt.

Untersuchung der Wirksamkeit von Sorbinsäure auf Bakterien und der Effekte von Sorbinsäure auf die Phagocytose und das intracelluläre Abtöten von Bakterien durch polymorphonucleare Leukozyten:

Bei diesen Untersuchungen wurden Sorbinsäurelösungen im Kulturmedium M199 verwendet. Sorbinsäure ist in diesem Medium M199 unter Verwendung von Wärme, wie sie zum Beispiel durch einen Mikrowellenofen erzeugt wird, leicht löslich. Bei diesen Untersuchungen wurden Konzentrationen der Sorbinsäure von 0,05 % bis 0,2 % (Gewicht/Volumen) verwendet. Als Kontrollmedium wurde das sorbinsäurefreie Kulturmedium M199 eingesetzt.

Die Untersuchung des Effektes von Sorbinsäure auf die Proliferation von Bakterien wurde an E. coli-ON2, einem gut definierten Stamm von E. coli, durchgeführt. Die bei dieser Untersuchung erhaltenen Ergebnisse sind in der nachfolgenden Tabelle 1 zusammengestellt und in den Figuren 1 und 2 graphisch dargestellt. Sowohl aus der Tabelle 1 als auch aus den Figuren 1 und 2 ist ersichlich, daß die Sorbinsäure gegenüber E. coli-ON2 bakteriostatisch wirksam ist, wobei diese Wirksamkeit bei pH 5,3 größer als bei pH 6,3 ist.

Der Effekt der Sorbinsäuren auf die Phagocytose und das intracelluläre Abtöten durch polymorphonucleare Leukozyten wurde unter Verwendung des gleichen E. coli-Stammes und anhand von menschlichen polymorphonuclearen Leukozyten (PMN) untersucht. Es wurde ebenfalls das gleiche Kulturmedium, wie es für die vorstehenden Untersuchungen eingesetzt, verwendet. Die Messungen wurden nach 60-minütiger Inkubation von PMN mit E. coli-0N 2 vorgenommen, und zwar sowohl bei pH 5,3 als auch bei pH 7,3. Die bei diesen Untersuchungen erhaltenen Ergebnisse sind in der Tabelle 2 zusammengestellt und in den Figuren 3 und 4 graphisch dargestellt. Die Ergebnisse zeigen, daß durch die Verwendung von Sorbinsäure die bakterizide Wirksamkeit von PMN nur wenig beeinträchtigt wird. Die bakterizide Wirksamkeit der PMN bleibt, wenn auch in etwas reduziertem Umfang, auch bei pH 5,3 aufrechterhalten.

**Ansprüche**

1. Dialysier- und Spülflüssigkeit zur intraperitonealen Verabreichung, enthaltend Elektrolyte in physiologischen Mengen und wenigstens eine osmotisch wirksame Substanz, **gekennzeichnet durch** einen antimikrobiellen Gehalt an Sorbinsäure und einen pH-Wert von 4 bis 7.

2. Flüssigkeit nach Anspruch 1, **dadurch gekennzeichnet,** daß der Gehalt an Sorbinsäure zwischen 0,001 und 1 Gew.-% liegt.

3. Flüssigkeit nach Anspruch 1 oder 2, **dadurch gekennzeichnet,** daß der pH-Wert 5 bis 6 beträgt.

4. Flüssigkeit nach Anspruch 1 bis 3, **dadurch gekennzeichnet,** daß der pH-Wert durch Milchsäure oder Essigsäure und NaOH oder Salzen hiervon eingestellt worden ist.

5. Verwendung von Sorbinsäure zur Herstellung einer Elektrolyte in physiologischen Mengen und wenigstens eine osmotisch wirksame Substanz enthaltenden Dialysier- und Spülflüssigkeit zur intraperitonealen Verabreichung.

Tabelle 1:

Effekt von Sorbinsäure auf das Wachstum
von E.coli-0N2 in M199-Medium

| | Stdn. | Mittleres Wachstum von E. coli-0N2 (log CUF/ml) $\pm$ SEM[a] | |
|---|---|---|---|
| | | pH 5,3 | pH 6,3 |
| 0 % SBZ | 0 | 3.73 $\pm$ 0.03 | 3.95 $\pm$ 0.09 |
| | 1 | 3.84 $\pm$ 0.09 | 3.96 $\pm$ 0.08 |
| | 2 | 4.12 $\pm$ 0.13 | 4.39 $\pm$ 0.05 |
| | 4 | 5.60 $\pm$ 0.10 | 5.90 $\pm$ 0.05 |
| | 6 | 6.94 $\pm$ 0.08 | 7.33 $\pm$ 0.13 |
| 0,05 % SBZ | 0 | 3.73 $\pm$ 0.03 | 3.95 $\pm$ 0.09 |
| | 1 | 3.78 $\pm$ 0.06 | 4.07 $\pm$ 0.21 |
| | 2 | 3.78 $\pm$ 0.11 | 4.30 $\pm$ 0.17 |
| | 4 | 4.18 $\pm$ 0.16 | 5.68 $\pm$ 0.10 |
| | 6 | 5.03 $\pm$ 0.39 | 6.88 $\pm$ 0.23 |
| 0,10 % SBZ | 0 | 3.73 $\pm$ 0.03 | 3.95 $\pm$ 0.09 |
| | 1 | 3.76 $\pm$ 0.05 | 3.94 $\pm$ 0.06 |
| | 2 | 3.68 $\pm$ 0.06 | 4.18 $\pm$ 0.07 |
| | 4 | 3.77 $\pm$ 0.06 | 5.25 $\pm$ 0.15 |
| | 6 | 3.93 $\pm$ 0.09 | 6.03 $\pm$ 0.06 |
| 0,15 % SBZ | 0 | 3.73 $\pm$ 0.03 | 3.95 $\pm$ 0.09 |
| | 1 | 3.57 $\pm$ 0.10 | 3.84 $\pm$ 0.20 |
| | 2 | 3.65 $\pm$ 0.10 | 3.87 $\pm$ 0.14 |
| | 4 | 3.63 $\pm$ 0.06 | 4.51 $\pm$ 0.03 |
| | 6 | 3.77 $\pm$ 0.15 | 5.57 $\pm$ 0.10 |
| 0,20 % SBZ | 0 | 3.73 $\pm$ 0.03 | 3.95 $\pm$ 0.09 |
| | 1 | 3.68 $\pm$ 0.03 | 3.71 $\pm$ 0.11 |
| | 2 | 3.84 $\pm$ 0.05 | 3.94 $\pm$ 0.10 |
| | 4 | 3.64 $\pm$ 0.05 | 5.25 $\pm$ 0.39 |
| | 6 | 3.68 $\pm$ 0.03 | 5.48 $\pm$ 0.06 |

[a]: auf Basis von mindestens 3 separaten Experimenten

SBZ: Sorbinsäure

EP 0 337 420 A2

Fig. 1 (zu Tabelle 1)

Effekt von Sorbinsäure in M199-Medium auf das Wachstum von E. coli-ON2 (pH 5,3)

LOG (Bact/ml)

Legend:
- --- Kontrolle
- -+- 0,05%
- -*- 0,10%
- -○- 0,15%
- -*- 0,20%

t (Stdn.)

Fig. 2 (zu Tabelle 1)

Effekt von Sorbinsäure in M199-Medium auf das Wachstum von E. coli-ON2 (pH 6,3)

LOG (Bact/ml)

t (Stdn.)

Legende:
- -- Kontrolle
- + 0,05%
- * 0,10%
- o 0,15%
- * 0,20%

EP 0 337 420 A2

Tabelle 2:

Effekt von Sorbinsäure auf die Phagocytose und das
intracelluläre Abtöten von E. coli-0N2 durch
polymorphonucleare Leukocyten

| pH | SBZ (%) in M199 | Phagocytose (%) [a] | Abtöten (%) [b] |
|----|----|----|----|
| 5,3 | 0 | 66 ± 5 | 96 ± 1 |
| | 0,05 | 59 ± 5 | 88 ± 3 |
| | 0,10 | 62 ± 5 | 65 ± 13 |
| | 0,15 | 56 ± 5 | 63 ± 12 |
| | 0,20 | 52 ± 7 | 60 ± 19 |
| 7,3 | 0 | 71 ± 4 | 98 ± 1 |
| | 0,05 | 64 ± 6 | 99 |
| | 0,10 | 60 ± 6 | 97 ± 1 |
| | 0,15 | 52 ± 4 | 97 ± 1 |
| | 0,20 | 50 ± 7 | 96 ± 1 |

a: Die Ergebnisse sind ausgedrückt als Mittelwerte ± SEM
und basieren auf zehn getrennten Experimenten, die im
Duplikat durchgeführt wurden.

b: Die Ergebnisse sind ausgedrückt als Mittelwerte ± SEM
und basieren auf drei getrennten Experimenten, die im
Duplikat durchgeführt wurden.

Fig. 3 (zu Tabelle 2)

Effekt von Sorbinsäure auf die Aufnahme von E. coli-ON2 durch PMN

Aufnahme (%)

PH

EP 0 337 420 A2

Fig. 4 (zu Tabelle 2)

**Effekt von Sorbinsäure auf das Abtöten von E. coli-ON2 durch PMN**

Abtöten (%)

Legende:
- 0% SBZ
- 0,05% SBZ
- 0,10% SBZ
- 0,15% SBZ
- 0,20% SBZ

PH: 5,3    7,3

EP 0 337 420 A2